# EUROPEAN PATENT SPECIFICATION

(11) **EP 1 863 495 B1**
(45) Date of publication and mention of the grant of the patent: **18.02.2015**
(21) Application number: 06738715.9
(22) Date of filing: 17.03.2006
(51) Int. Cl.: A01N 25/00, A01N 65/00, A61P 35/00, A61K 31/6615

(54) **CALCIUM AND SODIUM SALT OF MYO-INOSITOL 1,6:2,3:4,5 TRIPYROPHOSPHATE FOR TREATING CANCER**
KALZIUMS- UND NATRIUMSSALZ VON MYO-INOSITOL 1,6:2,3:4,5-TRIPYROPHOSPHAT ZUR BEHANDLUNG VON KREBS
SEL DE CALCIUM ET DE SODIUM DE MYO-INOSITOL 1,6:2,3:4,5 TRIPYROPHOSPHATE POUR TRAITER LE CANCER

(30) Priority: 18.03.2005 US 663491 P
(43) Date of publication of application: 12.12.2007
(73) Proprietor: NormOxys, Inc., Boston, MA 02135 (US)
(72) Inventor: NICOLAU, Claude, Newton, Massachusetts 02459 (US); LEHN, Jean-Marie, F-67000 Strasbourg (FR); FYLAKTAKIDOU, Konstantina, C., GR-55438 Thessaloniki (GR); GREFERATH, Ruth, 77694 Kehl (DE)
(74) Representative: Grund, Martin
(86) International application number: PCT/US2006/009682
(87) International publication number: WO 2006/102060

(56) References cited:
- WO-A1-03/092700
- WO-A1-03/092700
- WO-A1-2007/081315
- US-A1- 2004 014 642
- FYLAKTAKIDOU K C ET AL: "Inositol tripyrophosphate: a new membrane permeant allosteric effector of haemoglobin" 15 March 2005 (2005-03-15), BIOORGANIC & MEDICINAL CHEMISTRY LETTERS, PERGAMON, ELSEVIER SCIENCE, GB LNKD- DOI:10.1016/J.BMCL.2005.01.064, PAGE(S) 1605 - 1608 , XP4771154 ISSN: 0960-894X [retrieved on 2005-03-15] * figure 1 * * page 1608, column 1, paragraph 3 *
- FYLAKTAKIDOU K.C. ET AL.: 'Inositol Tripyrophosphate: A New Membrane Permeant Allosteric Effector of Haemoglobin' BIOORGANIC & MEDICINAL CHEMISTRY LETTERS vol. 15, no. 6, 15 March 2005, pages 1605 - 1608, XP004771154
- KLEDA ET AL: "Suppression of hypoxia induced HIF-1alpha...", PNAS, vol. 103, no. 42, 2006,

## Description

This application claims the benefit under 35 USC 119(e) to US Provisional Application 60/663,491 filed March 18, 2005.

### FIELD OF THE INVENTION

The present invention is directed to compositions for using the calcium salt of inositol-tripyrophosphate (ITPP-Ca) to enhance oxygen delivery by red blood. ITPP-Ca is an allosteric effector of hemoglobin which has the ability to cross the plasma membrane of red blood cells and lower the oxygen affinity of the hemoglobin of red blood cells. The present invention is further directed to the use of ITPP-Ca to inhibit angiogenesis and enhance radiation sensitivity of hypoxic tumors. The present invention is further directed to the use of ITPP-Ca to enhance PO₂ in hypoxic tumors.

### BACKGROUND OF THE INVENTION

In the vascular system of an adult human being, blood has a volume of about 5 to 6 liters. Approximately one half of this volume is occupied by cells, including red blood cells (erythrocytes), white blood cells (leukocytes), and blood platelets. Red blood cells comprise the majority of the cellular components of blood. Plasma, the liquid portion of blood, is approximately 90 percent water and 10 percent various solutes. These solutes include plasma proteins, organic metabolites and waste products, and inorganic compounds.

The major function of red blood cells is to transport oxygen from the lungs to the tissues of the body, and transport carbon dioxide from the tissues to the lungs for removal. Very little oxygen is transported by the blood plasma because oxygen is only sparingly soluble in aqueous solutions. Most of the oxygen carried by the blood is transported by the hemoglobin of the erythrocytes. Erythrocytes in mammals do not contain nuclei, mitochondria or any other intracellular organelles, and they do not use oxygen in their own metabolism. Red blood cells contain about 35 percent by weight hemoglobin, which is responsible for binding and transporting oxygen.

Hemoglobin is a protein having a molecular weight of approximately 64,500 daltons. It contains four polypeptide chains and four heme prosthetic groups in which iron atoms are bound in the ferrous state. Normal globin, the protein portion of the hemoglobin molecule, consists of two alpha chains and two beta chains. Each of the four chains has a characteristic tertiary structure in which the chain is folded. The four polypeptide chains fit together in an approximately tetrahedral arrangement, to constitute the characteristic quaternary structure of hemoglobin. There is one heme group bound to each polypeptide chain which can reversibly bind one molecule of molecular oxygen. When hemoglobin combines with oxygen, oxyhemoglobin is formed. When oxygen is released, the oxyhemoglobin is reduced to deoxyhemoglobin.

Delivery of oxygen to tissues, including tumors, depends upon a number of factors including, but not limited to, the volume of blood flow, the number of red blood cells, the concentration of hemoglobin in the red blood cells, the oxygen affinity of the hemoglobin and, in certain species, on the molar ratio of intraerythrocytic hemoglobins with high and low oxygen affinity. The oxygen affinity of hemoglobin depends on four factors as well, namely: (1) the partial pressure of oxygen; (2) the pH; (3) the concentration of 2,3-diphosphoglycerate (DPG) in the hemoglobin; and (4) the concentration of carbon dioxide. In the lungs, at an oxygen partial pressure of 100 mm Hg, approximately 98% of circulating hemoglobin is saturated with oxygen. This represents the total oxygen transport capacity of the blood. When fully oxygenated, 100 ml of whole mammalian blood can carry about 21 ml of gaseous oxygen.

The effect of the partial pressure of oxygen and the pH on the ability of hemoglobin to bind oxygen is best illustrated by examination of the oxygen saturation curve of hemoglobin. An oxygen saturation curve plots the percentage of total oxygen-binding sites of a hemoglobin molecule that are occupied by oxygen molecules when solutions of the hemoglobin molecule are in equilibrium with different partial pressures of oxygen in the gas phase.

The oxygen saturation curve for hemoglobin is sigmoid. Thus, binding the first molecule of oxygen increases the affinity of the remaining hemoglobin for binding additional oxygen molecules. As the partial pressure of oxygen is increased, a plateau is approached at which each of the hemoglobin molecules is saturated and contains the upper limit of four molecules of oxygen.

The reversible binding of oxygen by hemoglobin is accompanied by the release of protons, according to the equation:

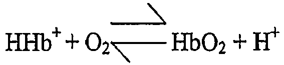

Thus, an increase in the pH will pull the equilibrium to the right and cause hemoglobin to bind more oxygen at a given partial pressure. A decrease in the pH will decrease the amount of oxygen bound.

In the lungs, the partial pressure of oxygen in the air spaces is approximately 90 to 100 mm Hg and the pH is also high relative to normal blood pH (up to 7.6). Therefore, hemoglobin will tend to become almost maximally saturated with oxygen in the lungs. At that pressure and pH, hemoglobin is approximately 98 percent saturated with oxygen. On the other hand, in the capillaries in the interior of the peripheral tissues, the partial pressure of oxygen is only about 25 to 40 mm Hg and the pH is also nearly neutral (about 7.2 to 7.3). Because muscle cells use oxygen at a high rate, thereby lowering the local concentration of oxygen, the release of some of the bound oxygen to the tissue is favored. As the blood passes through the capillaries in the muscles, oxygen will be released from the nearly saturated hemoglobin in the red blood cells into the blood plasma and then into the muscle cells. Hemoglobin will release about a fourth of its bound oxygen as it passes through the muscle capillaries, so that when it leaves the muscle, it will be only about 75 percent saturated. In general, the hemoglobin in the venous blood leaving the tissue cycles between about 65 and 97 percent saturation with oxygen in its repeated circuits between the lungs and the peripheral tissues. Thus, oxygen partial pressure and pH function together to effect the release of oxygen by hemoglobin.

A third important factor in regulating the degree of oxygenation of hemoglobin is the allosteric effector 2,3-diphosphoglycerate (DPG). DPG is the normal physiological effector of hemoglobin in mammalian erythrocytes. DPG regulates the oxygen-binding affinity of hemoglobin in the red blood cells in relationship to the oxygen partial pressure in the lungs. The higher the concentration of DPG in the cell, the lower the affinity of hemoglobin for oxygen.

When the delivery of oxygen to the tissues is chronically reduced, the concentration of DPG in the erythrocytes is higher than in normal individuals. For example, at high altitudes the partial pressure of oxygen is significantly less. Correspondingly, the partial pressure of oxygen in the tissues is less. Within a few hours after a normal human subject moves to a higher altitude, the DPG level in the red blood cells increases, causing more DPG to be bound and the oxygen affinity of the hemoglobin to decrease. Increases in the DPG level of red cells also occur in patients suffering from hypoxia. This adjustment allows the hemoglobin to release its bound oxygen more readily to the tissues to compensate for the decreased oxygenation of hemoglobin in the lungs. The reverse change occurs when people are acclimated to high altitudes and descend to lower altitudes.

As normally isolated from blood, hemoglobin contains a considerable amount of DPG. When hemoglobin is "stripped" of its DPG, it shows a much higher affinity for oxygen. When DPG is increased, the oxygen binding affinity of hemoglobin decreases. A physiologic allosteric effector such as DPG is therefore essential for the normal release of oxygen from hemoglobin in the tissues.

While DPG is the normal physiologic effector of hemoglobin in mammalian red blood cells, phosphorylated inositols are found to play the same role in the erythrocytes of some birds and reptiles. Although inositol hexaphosphate (IHP) is unable to pass through the mammalian erythrocyte membrane, it is capable of combining with hemoglobin of mammalian red blood cells at the binding site of DPG to modify the allosteric conformation of hemoglobin, the effect of which is to reduce the affinity of hemoglobin for oxygen. For example, DPG can be replaced by IHP, which is far more potent than DPG in reducing the oxygen affinity of hemoglobin. IHP has a 1000-fold higher affinity to hemoglobin than DPG (R. E. Benesch et al., Biochemistry, Vol. 16, pages 2594-2597 (1977)) and increases the P₅₀ of hemoglobin up to values of 96.4 mm, Hg at pH 7.4, and 37 degrees C. (J. Biol. Chem., Vol. 250, pages 7093-7098 (1975)).

The oxygen release capacity of mammalian red blood cells can be enhanced by introducing certain allosteric effectors of hemoglobin into erythrocytes, thereby decreasing the affinity of hemoglobin for oxygen and improving the oxygen economy of the blood. This phenomenon suggests various medical applications for treating individuals who are experiencing lowered oxygenation of their tissues due to the inadequate function of their lungs or circulatory system.

Because of the potential medical benefits to be achieved from the use of these modified erythrocytes, various techniques have been developed in the prior art to enable the encapsulation of allosteric effectors of hemoglobin in erythrocytes. Accordingly, numerous devices have been designed to assist or simplify the encapsulation procedure. The encapsulation methods known in the art include osmotic pulse (swelling) and reconstitution of cells, controlled lysis and resealing, incorporation of liposomes, and electroporation. Current methods of electroporation make the procedure commercially impractical on a scale suitable for commercial use.

The following references describe the incorporation of polyphosphates into red blood cells by the interaction of liposomes loaded with IHP: Gersonde, et al., "Modification of the Oxygen Affinity of Intracellular Hemoglobin by Incorporation of Polyphosphates into Intact Red Blood Cells and Enhanced O2 Release in the Capillary System", Biblthca. Haemat., No. 46, pp. 81-92 (1980); Gersonde, et al., "Enhancement of the O2 Release Capacity and of the Bohr-Effect of Human Red Blood Cells after Incorporation of Inositol Hexaphosphate by Fusion with Effector-Containing Lipid Vesicles", Origins of Cooperative Binding of Hemoglobin (1982); and Weiner, "Right Shifting of Hb-O2 Dissociation in Viable Red Cells by Liposomal Technique," Biology of the Cell, Vol. 47, (1983).

Additionally, U.S. Pat. Nos. 4,192,869, 4,321,259, and 4,473,563 to Nicolau et al. describe a method whereby fluid-charged lipid vesicles are fused with erythrocyte membranes, depositing their contents into the red blood cells. In this manner, it is possible to transport allosteric effectors, such as IHP into erythrocytes, where due to its much higher binding constant IHP replaces DPG at its binding site in hemoglobin.

In accordance with the liposome technique, IHP is dissolved in a phosphate buffer until the solution is saturated and a mixture of lipid vesicles is suspended in the solution. The suspension is then subjected to ultrasonic treatment or an injection process, and then centrifuged. The upper suspension contains small lipid vesicles containing IHP, which are then collected. Erythrocytes are added to the collected suspension and incubated, during which time the lipid vesicles containing IHP fuse with the cell membranes of the erythrocytes, thereby depositing their contents into the interior of the erythrocyte. The modified erythrocytes are then washed and added to plasma to complete the product.

The drawbacks associated with the liposomal technique include poor reproducibility of the IHP concentrations incorporated in the red blood cells and significant hemolysis of the red blood cells following treatment. Additionally, commercialization is not practical because the procedure is tedious and complicated.

In an attempt to solve the drawbacks associated with the liposomal technique, a method of lysing and the resealing red blood cells was developed. This method is described in the following publication: Nicolau, et al., "Incorporation of Allosteric Effectors of Hemoglobin in Red Blood Cells. Physiologic Effects," Biblthca. Haemat., No. 51, pp. 92-107, (1985). Related U.S. Pat. Nos. 4,752,586 and 4,652,449 to Ropars et al. also describe a procedure of encapsulating substances having biological activity in human or animal erythrocytes by controlled lysis and resealing of the erythrocytes, which avoids the red blood cell-liposome interactions.

The technique is best characterized as a continuous flow dialysis system, which functions in a manner similar to the osmotic pulse technique. Specifically, the primary compartment of at least one dialysis element is continuously supplied with an aqueous suspension of erythrocytes, while the secondary compartment of the dialysis element contains an aqueous solution which is hypotonic with respect to the erythrocyte suspension. The hypotonic solution causes the erythrocytes to lyse. The erythrocyte lysate is then contacted with the biologically active substance to be incorporated into the erythrocyte. To reseal the membranes of the erythrocytes, the osmotic and/or oncotic pressure of the erythrocyte lysate is increased and the suspension of resealed erythrocytes is recovered.

In related U.S. Pat. Nos. 4,874,690 and 5,043,261 to Goodrich et al., a related technique involving lyophilization and reconstitution of red blood cells is disclosed. As part of the process of reconstituting the red blood cells, the addition of various polyanions, including IHP, is described. Treatment of the red blood cells according to the process disclosed results in a cell with unaffected activity. Presumably, the IHP is incorporated into the cell during the reconstitution process, thereby maintaining the activity of the hemoglobin.

In U.S. Pat. Nos. 4,478,824 and 4,931,276 to Franco et al., a second related method and apparatus is described for introducing effectively non-ionic agents, including IHP, into mammalian red blood cells by effectively lysing and resealing the cells. The procedure is described as the "osmotic pulse technique." In practicing the osmotic pulse technique, a supply of packed red blood cells is suspended and incubated in a solution containing a compound which readily diffuses into and out of the cells, the concentration of the compound being sufficient to cause diffusion thereof into the cells so that the contents of the cells become hypertonic. Next, a trans-membrane ionic gradient is created by diluting the solution containing the hypertonic cells with an essentially isotonic aqueous medium in the presence of at least one desired agent to be introduced, thereby causing diffusion of water into the cells with a consequent swelling and an increase in permeability of the outer membranes of the cells. This "osmotic pulse" causes the diffusion of water into the cells and a resultant swelling of the cells which increase the permeability of the outer cell membrane to the desired agent. The increase in permeability of the membrane is maintained for a period of time sufficient only to permit transport of at least one agent into the cells and diffusion of the compound out of the cells.

Polyanions which may be used in practicing the osmotic pulse technique include pyrophosphate, tripolyphosphate, phosphorylated inositols, 2,3-diphosphoglycerate (DPG), adenosine triphosphate, heparin, and polycarboxylic acids which are water-soluble, and non-disruptive to the lipid outer bilayer membranes of red blood cells.

The osmotic pulse technique has several shortcomings including low yield of encapsulation, incomplete resealing, loss of cell content and a corresponding decrease in the life span of the cells. The technique is tedious, complicated and unsuited to automation. For these reasons, the osmotic pulse technique has had little commercial success.

Another method for encapsulating various biologically-active substances in erythrocytes is electroporation. Electroporation has been used for encapsulation of foreign molecules in different cell types, including IHP in red blood cells, as described in Mouneimne, et al., "Stable rightward shifts of the oxyhemoglobin dissociation curve induced by encapsulation of inositol hexaphosphate in red blood cells using electroporation," FEBS, Vol. 275, No. 1, 2, pp. 117-120 (1990). Also, see U.S. Patent No. 5,612,207.

Angiogenesis is the generation of new blood vessels into a tissue or organ and is related to oxygen tension in the tissues. Under normal physiological conditions, humans and animals undergo angiogenesis only in very specific, restricted situations. For example, angiogenesis is normally observed in wound healing, fetal and embryonal development, and formation of the corpus luteum, endometrium and placenta.

Angiogenesis is controlled through a highly regulated system of angiogenic stimulators and inhibitors. The control of angiogenesis is altered in certain disease states and, in many cases, pathological damage associated with the diseases is related to uncontrolled angiogenesis. Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. Endothelial cells, lining the lumen of blood vessels, then protrude through the basement membrane. Angiogenic stimulants induce the endothelial cells to migrate through the eroded basement membrane. The migrating cells form a "sprout" off the parent blood vessel where the endothelial cells undergo mitosis and proliferate. The endothelial sprouts merge with each other to form capillary loops, creating a new blood vessel.

Persistent, unregulated angiogenesis occurs in many disease states, tumor metastases, and abnormal growth by endothelial cells. The diverse pathological disease states in which unregulated angiogenesis is present have been grouped together as angiogenic-dependent or angiogenic-associated diseases.

The hypothesis that tumor growth is angiogenesis-dependent was first proposed in 1971. (Folkman, New Eng. J. Med., 285:1182-86 (1971)). In its simplest terms, this hypothesis states: "Once tumor 'take' has occurred, every increase in tumor cell population must be preceded by an increase in new capillaries converging on the tumor." Tumor 'take' is currently understood to indicate a prevascular phase of tumor growth in which a population of tumor cells occupying a few cubic millimeters volume, and not exceeding a few million cells, can survive on existing host microvessels. Expansion of tumor volume beyond this phase requires the induction of new capillary blood vessels. For example, pulmonary micrometastases in the early prevascular phase in mice would be undetectable except by high power microscopy on histological sections.

Angiogenesis has been associated with a number of different types of cancer, including solid tumors and blood-borne tumors. Solid tumors with which angiogenesis has been associated include, but are not limited to, rhabdomyosarcomas, retinoblastoma, Ewing's sarcoma, neuroblastoma, and osteosarcoma. Angiogenesis is also associated with blood-borne tumors, such as leukemias, any of various acute or chronic neoplastic diseases of the bone marrow in which unrestrained proliferation of white blood cells occurs, usually accompanied by anemia, impaired blood clotting, and enlargement of the lymph nodes, liver and spleen. It is believed that angiogenesis plays a role in the abnormalities in the bone marrow that give rise to leukemia tumors and multiple myeloma diseases.

One of the most frequent angiogenic diseases of childhood is the hemangioma. A hemangioma is a tumor composed of newly formed blood vessels. In most cases, the tumors are benign and regress without intervention. In more severe cases, the tumors progress to large cavernous and infiltrative forms and create clinical complications. Systemic forms of hemangiomas, hemangiomatoses, have a high mortality rate. Therapy-resistant hemangiomas exist that cannot be treated with therapeutics currently in use.

Another angiogenesis associated disease is rheumatoid arthritis. The blood vessels in the synovial lining of the joints undergo angiogenesis. In addition to forming new vascular networks, the endothelial cells release factors and reactive oxygen species that lead to pannus growth and cartilage destruction. Angiogenesis may also play a role in osteoarthritis. The activation of the chondrocytes by angiogenic-related factors contributes to the destruction of the joint. At a later stage, the angiogenic factors promote new bone growth. Therapeutic intervention that prevents the cartilage destruction could halt the progress of the disease and provide relief for persons suffering with arthritis.

Chronic inflammation may also involve pathological angiogenesis. Such diseases as ulcerative colitis and Crohn's disease show histological changes with the ingrowth of new blood vessels into inflamed tissues. Bartonelosis, a bacterial infection found in South America, can result in a chronic stage that is characterized by proliferation of vascular endothelial cells. Another pathological role associated with angiogenesis is found in atherosclerosis. The plaques formed within the lumen of blood vessels have been shown to have angiogenic stimulatory activity.

As mentioned above, several lines of evidence indicate that angiogenesis is essential for the growth and persistence of solid tumors and their metastases. Once angiogenesis is stimulated, tumors upregulate the production of a variety of angiogenic factors, including fibroblast growth factors (aFGF and bFGF) and vascular endothelial growth factor/vascular permeability factor (VEGF/VPF) [2,3].

The role of VEGF in the regulation of angiogenesis has been the object of intense investigation [5-10]. Whereas VEGF represents a critical, rate-limiting step in physiological angiogenesis, it appears to be also important in pathological angiogenesis, such as that associated with tumor growth [H]. VEGF is also known as vascular permeability factor, based on its ability to induce vascular leakage [13]. Several solid tumors produce ample amounts of VEGF, which stimulates proliferation and migration of endothelial cells, thereby inducing neovascularization [12,13]. VEGF expression has been shown to significantly affect the prognosis of different kinds of human cancer. Oxygen tension in the tumor has a key role in regulating the expression of VEGF gene. VEGF mRNA expression is induced by exposure to low oxygen tension under a variety of pathophysiological circumstances [13]. Growing tumors are characterized by hypoxia, which induces expression of VEGF and may also be a predictive factor for the occurrence of metastatic disease.

What is needed, therefore, is a substantially non-toxic composition that can regulate oxygen tension in the tissue, especially a tumor. In addition, what is needed is a simple and easily administered, preferably orally, composition that is capable of causing significant right shifts of the P₅₀ value for red blood cells.

### SUMMARY OF THE INVENTION

The present invention provides a pharmaceutical composition comprising the mixture of the sodium and calcium salt of myo-inositol tripyrophosphate and a pharmaceutically acceptable adjuvant, diluent, carrier, on excipient thereof, for use in the treatment of cancer. Cancers that can be treated by the present invention include, breast cancer, prostrate cancer, renal cell cancer, brain cancer, ovarian cancer, colon cancer, bladder cancer, pancreatic cancer, stomach cancer, esophageal cancer, cutaneous melanoma, liver cancer, lung cancer, testicular cancer, kidney cancer, bladder cancer, cervical cancer, lymphoma, parathyroid cancer, penile cancer, rectal cancer, small intestine cancer, thyroid cancer, uterine cancer, Hodgkin's lymphoma, lip and oral cancer, skin cancer, leukemia or multiple myeloma.

These and other objects, features and advantages of the present invention will become apparent after a review of the following detailed description of the disclosed embodiments.

### BRIEF DESCRIPTION OF THE DRAWINGS

Figure 1 depicts the chemical structure of the calcium salt of inositol- tripyrophosphate (ITPP).
Figure 2 shows the time course of the induced right shift of the O₂- hemoglobin dissociation curve (ODC) in the mice ingesting ITPP for 4 days, as well as the absence of significant P₅₀ shifts in the control animals.
Figure 3 shows that the level of ions, such as sodium and potassium and calcium, were normal after oral application of ITPP in mice.
Figure 4 shows the relation of P50 shift [%] to number of erythrocytes/mm³ in mice having received ITPP.
Figure 5 demonstrates ITPP toleration by mice, up to a concentration of 150mM. The level of ions, such as sodium, potassium and calcium were normal after intraperitoneal (ip) injection.
Figure 6 shows an agarose gel indicating the VEGF mRNA concentrations in tumors from control and ITPP drinking animals.
Figure 7 shows the Western blot assay of the expressed VEGF in tumors of control and ITPP-treated Lewis Lung carcinoma (LLC) tumor-bearing animals.

### DETAILED DESCRIPTION OF THE INVENTION

The pharmaceutical composition of this invention may also contain, or be co-administered (simultaneously or sequentially) with, one or more pharmacological agents of value in treating cancer.

A person skilled in the art will be able by reference to standard texts, such as Remington's Pharmaceutical Sciences 17th edition, to determine how the formulations are to be made and how these may be administered.

By "an effective amount" as referred to in this specification, it is meant a therapeutically or prophylactically effective amount. Such amounts can be readily determined by an appropriately skilled person, taking into account the condition to be treated, the route of administration and other relevant factors. Such a person will readily be able to determine a suitable dose, mode and frequency of administration. "Individual" as referred to in this application refers to any animal that may be in need of treatment for a given condition. "Individual" includes humans, other primates, household pets, livestock, rodents, other mammals, and any other animal(s) that may typically be treated by a veterinarian.

The compositions described above can be provided as physiologically acceptable formulations using known techniques, and these formulations can be administered by standard routes. In general, the combinations may be administered by the topical, oral, rectal, intraperitoneal or parenteral (e.g., intravenous, subcutaneous or intramuscular) route. In addition, the combinations may be incorporated into polymers allowing for sustained release, the polymers being implanted in the vicinity of where delivery is desired, for example, at the site of a tumor, or into an a cavity or blood vessel that will lead to easy delivery to the place to be treated. The dosage of the composition will depend on the condition being treated, the particular derivative used, and other clinical factors such as weight and condition of the patient and the route of administration of the compound. However, for oral administration, a recommended dosage is in the range of 0.1 to 5.0 g/kg/day. A dosage for oral administration is in the range of 0.5 to 2.0 g/kg/day or alternatively, about 0.5 to about 1.5 g/kg/day. In an alternate embodiment, a dosage for oral administration is in the range of about 0.80 to 1.0 g/kg/day or alternatively, about between 0.9 to 1.1 g/kg/day.

The formulations in accordance with the present invention can be administered in the form of tablet, a capsule, a lozenge, a cachet, a solution, a suspension, an emulsion, a powder, an aerosol, a suppository, a spray, a pastille, an ointment, a cream, a paste, a foam, a gel, a tampon, a pessary, a granule, a bolus, a mouthwash, or a transdermal patch.

The formulations include those suitable for oral, rectal, nasal, inhalation, topical (including dermal, transdermal, buccal and sublingual), vaginal, parenteral (including subcutaneous, intramuscular, intravenous, intraperitoneal, intradermal, intraocular, intratracheal, and epidural) or inhalation administration. The formulations may conveniently be presented in unit dosage form and may be prepared by conventional pharmaceutical techniques. Such techniques include the step of bringing into association the active ingredient and a pharmaceutical carrier(s) or excipient(s). In general, the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil emulsion, etc.

A tablet may be made by compression or molding, optionally with one or more accessory ingredients. Compressed tablets may be prepared by compressing, in a suitable machine, the active ingredient in a free-flowing form such as a powder or granules, optionally mixed with a binder, lubricant, inert diluent, preservative, surface-active or dispersing agent. Molded tablets may be made by molding, in a suitable machine, a mixture of the powdered compound moistened with an inert liquid diluent. The tablets may optionally be coated or scored and may be formulated so as to provide a slow or controlled release of the active ingredient therein.

Formulations suitable for topical administration in the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutically acceptable carrier. A preferred topical delivery system is a transdermal patch containing the ingredient to be administered.

Formulations for rectal administration may be presented as a suppository with a suitable base comprising, for example, cocoa butter and/or a salicylate.

Formulations suitable for nasal administration, wherein the carrier is a solid, include a coarse powder having a particle size, for example, in the range of 20 to 500 microns which is administered in the manner in which snuff is taken; *i.e.,* by rapid inhalation through the nasal passage from a container of the powder held close up to the nose. Suitable formulations, wherein the carrier is a liquid, for administration, as for example, a nasal spray or as nasal drops, include aqueous or oily solutions of the active ingredient.

Formulations suitable for vaginal administration may be presented as pessaries, tampons, creams, gels, pastes, foams or spray formulations containing, in addition to the active ingredient, ingredients such as carriers as are known in the art to be appropriate.

Formulation suitable for inhalation may be presented as mists, dusts, powders or spray formulations containing, in addition to the active ingredient, ingredients such as carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in freeze-dried (lyophilized) conditions requiring only the addition of a sterile liquid carrier, for example, water for injections, immediately prior to use. Extemporaneous injection solutions and suspensions may be prepared from sterile powders, granules and tablets of the kinds previously described.

Preferred unit dosage formulations are those containing a daily dose or unit, daily sub-dose, as herein above recited, or an appropriate fraction thereof, of the administered ingredient.

It should be understood that in addition to the ingredients, particularly those mentioned above, the formulations of the present invention may include other agents conventional in the art having regard to the type of formulation in question, for example, those suitable for oral administration may include flavoring agents or other agents to make the formulation more palatable and more easily swallowed.

### Experimental

For the *in vitro* experiments, ITPP was dissolved in deionized water, pH was adjusted at pH 7 and, for incubation with whole blood, the osmolarity of the ITPP solutions was adjusted with glucose to 270-297 mOsM. Mixtures of hemoglobin and ITPP were measured with a HEMOX analyzer (PD Marketing, London) immediately after mixing. Red blood cells were incubated with ITPP for 1 hour at 37°C. Following incubation, the cells were washed 3 times with Bis-Tris- buffer (pH=7.0) and then used for P₅₀ measurement.

In experiments conducted in vivo in which ITPP was administered orally, a significant shift of the P₅₀ value of circulating RBCs was observed. ITPP was dissolved in drinking water at a 20g/L-concentration (= 27mM, pH ~7.0.) and offered for drinking ad libitum.

The following examples illustrate the invention.

### REFERENCE EXAMPLE 1

### Induced Right Shift of the O₂-Hemoglobin Dissociation Curve (ODC) in Mice (Orally Administered)

Twelve (12) C57BL/6 mice were fed an ITPP-solution (20 g/L- concentration = 27mM, pH ∼7.0) for 4 days (up to 25 ml per 24 hrs). Three (3) control mice drank pure water, and four (4) control mice were fed a solution of myo-inositol hexaphosphate (IHP) (same concentration and pH as ITPP). Blood was collected from all mice on day 0 (before treatment started), and on days 1, 2, 4, 6, 7, 8, 10, 11 and 12 (after treatment had started), in order to measure P₅₀ values.

### Results

Oral application of ITPP caused significant right shifts of P₅₀ (up to 31 %) in mice.

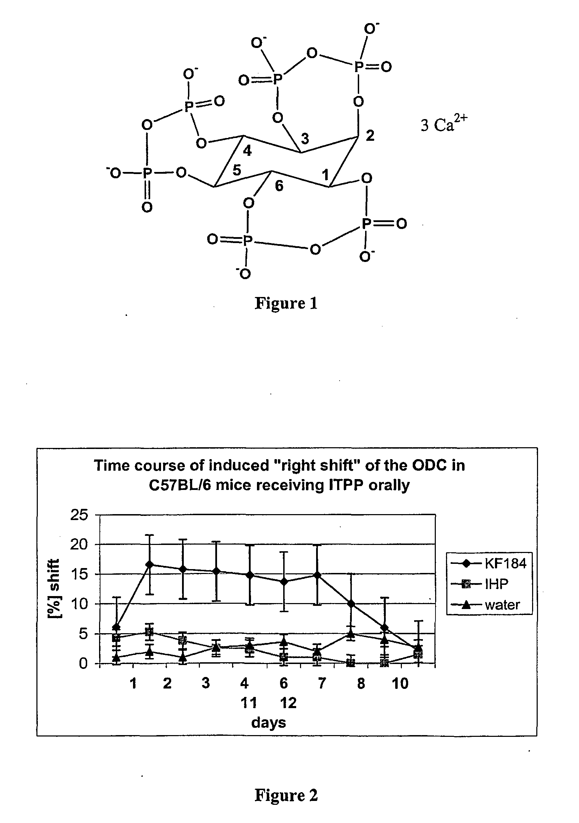

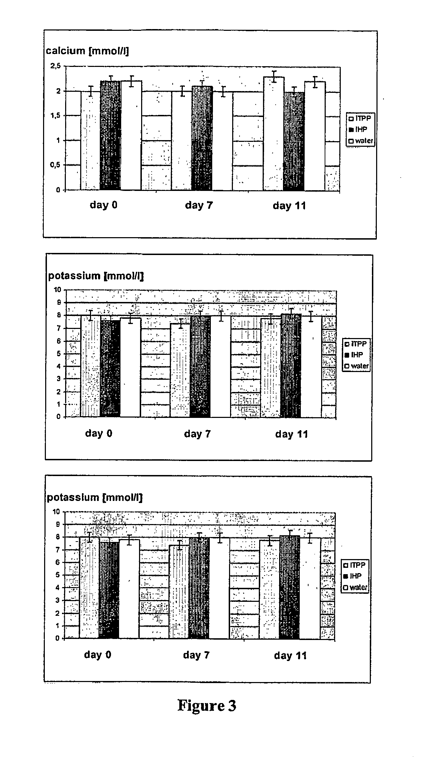

ITPP, when orally administered at a concentration of 27 mM, causes a right shift of the P₅₀ value in murine circulating red blood cells (see Figure 2). There is a time lag of approximately 48 hrs. before the maximum shift is attained. Maximal P₅₀ shifts are reached between day 2 and day 4, after beginning oral administration of ITPP. After 12 days, P₅₀ values are back to control values, when ingestion is stopped on day 4. There is a significant effect of ITPP ingestion on the number of red blood cells. Although not wishing to be bound by theory, it is believed that the effect of ITPP ingestion on the number of red blood cells wherein down-regulation of erythropoiesis is seen is due to the increased P₅₀. Hemolysis can be ruled out, as lysis of the red blood cells never occurred *in vitro.* The level of ions, such as sodium and potassium and calcium were normal after oral application of ITPP in mice (Figure 3). Figure 3 contains the mean values and SD for the serum concentration of sodium, potassium and calcium obtained on day 0, 7 and 11 after oral administration of ITPP (4 mice), IHP (3 mice) or water (3 mice).

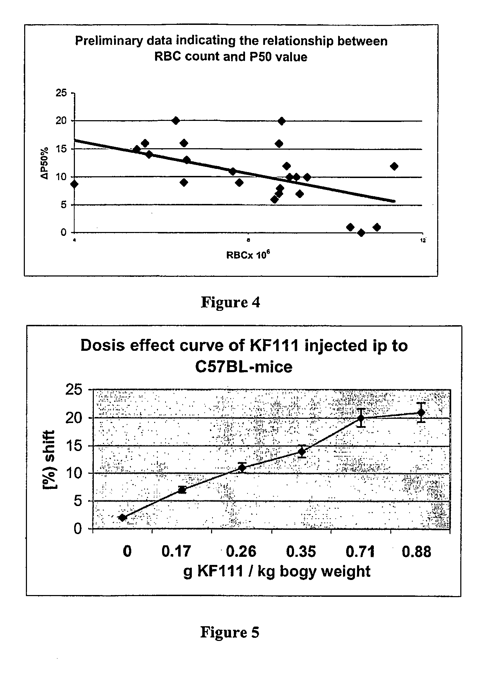

Blood counts were measured from all mice, on day 0, 7 and 11. The number of red blood cells in mice having ingested ITPP was reduced. There were no significant differences in the number of white blood cells (e.g. granulocytes, macrophages etc.) in blood from the mice in different groups. Figure 4 shows the RBC counts for mice with shifted ODC as compared to controls. Figure 4 further shows the relation of P₅₀ shift [%] to number of erythrocytes/mm³ in mice having received ITPP. It appears, based upon preliminary data,that an inverse relationship exists between the number of red blood cells and shift of their P50 value. The basal value of the red blood cell count is restored, once ΔP₅₀ becomes 0%, 12 days after ingestion of ITPP.

### REFERENCE EXAMPLE 2

### Induced Bright Shift of the ODC in Mice (Injected Intraperitoneally)

When ITPP (pH 7, 200 µl) was injected intraperitoneally in mice, the P₅₀ values of circulating red blood cells were shifted up to 23%. Figure 5 demonstrates that ITPP was well tolerated by mice, up to a concentration of 150 mM. The level of ions, such as sodium, potassium and calcium were normal after intraperitoneal injection. Six (6) mice were each injected intraperitoneally with 45 - 150 mM (= 0.17-0.88 g/kg body weight) of ITPP. The mean values of % shift and standard deviation are shown in Figure 5.

The concentration dependence of the P50 shifts induced by ITPP is an additional indication that this compound crosses the membrane of the red blood cells.

### REFERENCE EXAMPLE 3

### Induced Right Shift of the OCD in Piglets (Intravenously Injected)

ITPP was also injected intravenously (IV) in piglets. A right shift of P₅₀ was observed when the compound was injected at a 1 g/kg body weight dose.

In order to check possible side effects of ITPP, the level of calcium in the serum of the injected piglet was determined. A strong drop in the Ca²⁺ concentration in the animal's blood immediately after infusion indicated the possibility that ITPP, with 3 dissociated phosphate groups binding Ca²⁺, reduces its availability as free ion in the blood. One day after infusion, the concentration of Ca²⁺ in the piglets' blood was restored to the normal value. These results are shown in Table 1.

**Table 1: Ca "2+ concentration in the piglet's circulation blood**

| Sample taken | Ca2+ conc. [mmol/L] |
|---|---|
| Before injection | 2.38 |
| **10 min after completion of injection** | 1.73 |
| **24 hrs after injection** | 2.36 |

Based upon this observation, a CaC12 (equimolar to ITPP) solution was injected with the ITPP solution, so that the dissociated phosphate groups of ITPP were saturated. None of the side effects observed previously occurred. The level of calcium remained constant and the P50 shift was again approximately 20% of the basal value. The level of sodium and potassium ions was unchanged after intravenous injection of ITPP in piglets.

### REFERENCE EXAMPLE 4

### Effect of in vivo Lowering of Hemoglobin's Affinity for O₂ by ITPP on Intratumoral PO₂, Angio genesis and Expression of VEGF mRNA

ITPP, when administered orally, intravenously, or intraperitoneally, inhibits angiogenesis in growing tumors by enhancing the PO₂ in the forming tumors. Thirty (30) C57BL/6 mice received 20 g/L of ITPP orally until the P₅₀ value showed a shift of at least 20% above the control value. Thereafter, all animals received 1x10⁶ Lewis Lung carcinoma (LLC) cells, injected in the dorsal cavity. At different time points, the VEGF mRNA were assayed by RT-PCR in the tumors growing in both groups of mice.

Tumor tissue samples were ground in a RIPA lysis buffer (1% Nonidet p-40 detergent, 50 mM Tris pH 8.0, 137 mM NaCl, 10% glycerol) supplemented with protease inhibitor cocktail (Roche, Reinach, Switzerland). After centrifugation for 10 minutes at 4°C and 12,000 g, protein concentrations of tissue extracts were determined according to the Bradford method. Detergent soluble protein samples (10 mg) were separated by size on a SDS-PAGE in 10% acrylamide gels and transferred to nitrocellulose membrane (Protran BA 85, Schleicher and Schuell, Dassel, Germany). Membranes were blocked for 3 hours at room temperature in 10% skim milk in Tris buffer saline containing 0.1% Tween, before an overnight incubation at 4°C with rabbit polyclonal antibodies recognizing human, mouse and rat vascular endothelial growth factor (VEGF A-20, sc-152, Santa Cruz Biotechnology, Santa Cruz, California) at a dilution of 1:200. Membranes were then probed for primary antibody with anti-rabbit (1:16,000) peroxidase conjugates (Sigma-Aldrich, L'Isle d'Abeau Chesnes, France) for 60 minutes at room temperature. The resulting complexes were visualized by enhanced chemiluminescence autoradiography (Amersham Pharma Biotech, Orsay, France).

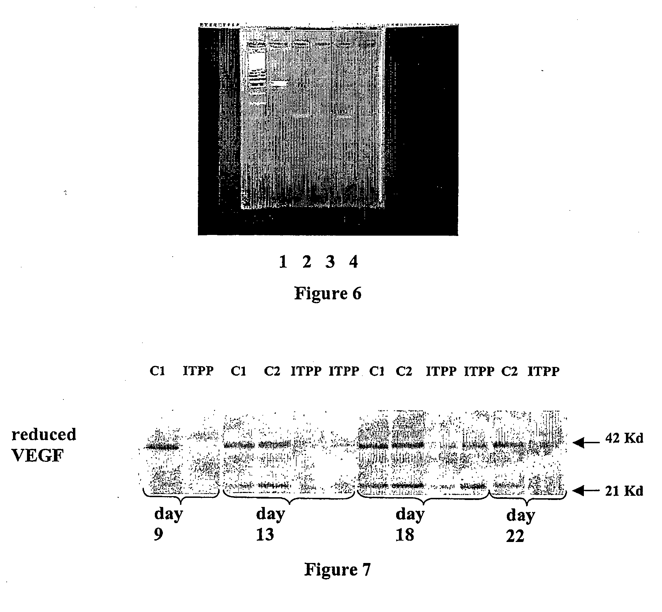

There was a difference in the level of mRNA of the VEGF gene in both groups. Figure 6 shows an agarose gel indicating the VEGF mRNA concentrations in tumors from control and ITPP drinking animals. The RT-PCR agarose gel assay of VEGF mRNAs from tumor tissue taken from 2 mice each on day 15 after inoculation of LLC cells (track 1: controls, track 2: ITPP treated animals) and day 30 after inoculation (track 3: control animals, track 4: ITPP treated animals). Figure 7 shows the Western blot assay of the expressed VEGF in tumors of control and ITPP-treated LLC tumor-bearing animals.

Quantification of the gel assays indicated a reduction by a factor of 10,000 of the amount of VEGF mRNAs detected in the tumors of animals having received ITPP, at day 9 and then, while differences remain between treated and untreated animals, they tend to decrease. This indicates that ITPP taken up by circulating red blood cells significantly increases tumor PO₂.

### EXAMPLE 5

### Effectiveness of the Calcium Salt of myo-inositol Tripyrophosphate

When *myo*-inositol tripyrophosphate-sodium salt (ITPP-Na) is mixed with CaCl₂, a mixture of ITPP-Na and ITPP-Ca (myo-inositol tripyrophosphate-calcium salt) is obtained. This mixture, when added to free hemoglobin or to whole blood induces a P₅₀ shift of 170% and 25%, respectively as shown in Tables 2 and 3 below. Please see the results in Tables 2 and 3 for compound 15. The compounds in Tables 2 and 3 are as follows: 4 is the pyridinium salt of ITPP, 5 is the sodium salt of ITPP (*i.e*., ITPP-Na), 7 is the N,N-dimethylcyclohexyl ammonium salt of ITPP, 11 is the cycloheptyl ammonium salt of ITPP, 12 is the cyclooctyl ammonium salt of ITPP, 13 is the piperazinium salt of ITPP, 14 is the tripiperazinium salt of ITPP, and 15 is the calcium salt of ITPP (*i.e.,* ITPP-Ca).

In Tables 2 and 3, the effectiveness of all of the salts of ITPP regarding their ability to act as allosteric effectors of hemoglobin can be seen. The sodium salt and the calcium salt of ITPP appear to be the best allosteric effectors for both free hemoglobin (Table 2) and in whole blood (Table 3). However, pigs injected intravenously with ITPP-Na at a rate of 1g/kg weight resulted in a number of adverse side effects. The intravenous injection of pigs with ITPP-Na resulted in flushing, an increase in the heart rate, and a decrease in the Ca²⁺ plasma concentration from 2.38 mmol/L to 1.76 mmol/L.

Administration of the mixture of the sodium and calcium salt of ITPP, at the same dosage did not induce any of the cited effects and the Ca²⁺ plasma concentration stayed unchanged at 2.38 mmol/L.

This lack of toxicity of the mixture of Na⁺ and Ca²⁺ ITPP salts induced the synthesis and purification of the ITPP Ca²⁺ salt, which is described below. While the Ca²⁺ ITPP salt was not quite the allosteric effector in pure hemoglobin or in red blood cells that the sodium salt was (see Tables 2 and 3), the calcium salt did not have any of the adverse side effects that were associated with the sodium salt when administered to one or more individuals. Accordingly, the calcium salt of ITPP was found to be of particular interest and was further studied.

### EXAMPLE 6

### Preparation of the Calcium Salt of myo-inositol 1,6:2.3:4,5-tripyrophosphate:

The hexasodium and hexapyridinium salts of *myo*-inositol tripyrophosphate (ITPP-Na and ITPP-py) are obtained from *myo*-inositol hexaphosphate (IHP) as described in K.C. Fylaktakidou, J. M. Lehn, R. Greferath and C. Nicolau, Bioorganic & Medicinal Chemistj Chemistry Letters, 2005, 15, 1605-1608. Other salts of *myo*-inositol tripyrophosphate can also be made in accordance with the Fylaktakidou et al. reference. See also, L. F. Johnson and M. E. Tate, Can. J. Chem., 1969, 47, 63, for a description of phytins.

Other compounds can be made from the above compounds. For example, passing an aqueous solution of ITPP-py over an ion-exchange Dowex H⁺ column gives a solution of the corresponding perprotonated form of *myo*-inositol tripyrophosphate (i.e., ITPP-H).

Treatment of the ITPP-H with three equivalents of calcium hydroxide (one equivalent per pyrophosphate group) yields the tricalcium salt ITPP-Ca, which can then be isolated by evaporation of the aqueous solution under reduced pressure such as by use of a rotary evaporator (*i.e.,* a rotovap).

Alternatively, ITPP-Ca can be produced by the addition of equimolar amounts of CaCl₂ with an aqueous solution of ITPP-Na. The resulting mixture gives ITPP-Ca, which is contaminated with NaCl.

The present invention relates to a pharmaceutical composition for use in the treatment of cancer comprising the calcium salt of myo-inositol tripyrophosphate and a pharmaceutically acceptable adjuvant, diluent, carrier, or excipient thereof. In this pharmaceutical composition, the inositol tripyrophosphate is optionally myoinositol 1,6:2,3:4,5 tripyrophosphate. The composition of the present invention also contains the sodium salt of myo-inositol tripyrophosphate.

In an embodiment, the above compositions comprise as the myoinositol tripyrophosphate, myo-inositol 1,6:2,3:4,5 tripyrophosphate. The composition is prepared at a dosage to treat cancer. The treatable cancers include, but are not limited to, rhabdomyosarcomas, retinoblastoma, Ewing's sarcoma, neuroblastoma, and/or osteosarcoma. Moreover, the cancers to be treated may optionally include one or more of breast cancer, prostate cancer, renal cell cancer, brain cancer, ovarian cancer, colon cancer, bladder cancer, pancreatic cancer, stomach cancer, esophageal cancer, cutaneous melanoma, liver cancer, lung cancer, testicular cancer, kidney cancer, bladder cancer, cervical cancer, lymphoma, parathyroid cancer, penile cancer, rectal cancer, small intestine cancer, thyroid cancer, uterine cancer, Hodgkin's lymphoma, lip and oral cancer, skin cancer, leukemia, or multiple myeloma.

In an embodiment, the composition of the present invention is prepared in any of the above-enumerated ways of delivering a dosage of myo-inositol 1,6:2,3:4,5 tripyrophosphate (such as the calcium salt of this compound) so that between about 0.5 and 1.5 g/kg, and optionally between about 0.9 and 1.1 g/kg per day, is delivered in an effective amount.

A method of making the myo-inositol 1,6:2,3:4,5 tripyrophosphate calcium salt comprises adding a calcium salt containing organic compound to a perprotonated form of myo-inositol tripyrophosphate. The calcium salt containing organic compound may be one or more of calcium hydroxide, calcium chloride, calcium bromide, calcium iodide, and calcium fluoride. The method may comprise adding at least a three to one ratio of the calcium containing organic compound relative to the perprotonated myo-inositol tripyrophosphate compound amount. The method may comprise adding at least a three to one ratio of the calcium hydroxide relative to the amount of perprotonated myo-inositol tripyrophosphate compound.

In another embodiment, the composition for use in treating cancer is for administration to an individual in a pharmaceutically acceptable amount of any of the above enumerated compositions, wherein the active ingredient in the composition (i.e., ITPP) is administered to an individual at a dosage of about 0.5 and 1.5 g/kg or alternatively, in an amount that is between about 0.9 and 1.1 g/kg per day.

It should be understood that any of the above described one or more elements from any embodiment can be combined with any one or more element in any other embodiment. Moreover, when a range is mentioned, it should be understood that it is contemplated that any real number that falls within the range is a contemplated end point. For example, if a range of 0.9 and 1.1 g/kg is given, it is contemplated that any real number value that falls within that range (for example, 0.954 to 1.052 g/kg) is contemplated as a subgenus range of the invention, even if those values are not explicitly mentioned. Finally, the invention should rather be defined by the below claims.

**Table 2. P₅₀ values of free Hb after incubation with compounds 4, 5, 7, 11-14 and 15, in vitro**

| Compound | *P₅₀* (Torr) free Hb | *P₅₀* (Torr) Hb + compound | *P₅₀* increase (%) + SD |
|---|---|---|---|
| **4** | **(H)** 15.3 | 31.6 | 107 ± 22 |
| | **(M)** 25.0 | 50.0 | 100 ± 18 |
| **5** | **(H)** 15.3 | 49.8 | 225 ± 19 |
| | **(M)** 24.9 | 69.7 | 180 ± 25 |
| | **(P)** 22.0 | 68.1 | 209 ± 39 |
| **7** | **(M)** 24.9 | 45.1 | 81 ± 15 |
| **11** | **(M)** 24.9 | 43.8 | 76 ± 13 |
| **12** | **(M)** 24.9 | 30.6 | 23 ± 5 |
| **13** | **(M)** 23.4 | 67.7 | 189 ± 43 |
| **14** | **(M)** 23.4 | 82.9 | 254 ± 49 |
| **15** | **(H)** 12.3 | 33.1 | 170 ± 32 |
| | **(M)** 26.9 | 61.9 | 130 ± 30 |

| | | | |
|---|---|---|---|
| **H** = human; **M** = murine; **P** = porcine free **Hb**. Concentration of the compound solution was **60** m**M**. Means of *P₅₀* shifts in % are shown. **SD** = standard deviation. Compounds 4, 7,11, **12, 14** and **15:** three *P₅₀* values each were used for the calculation of means; compound **5**: with human blood: five values, murine blood: ten values and porcine blood: three values were used for the calculation of the means *of P₅₀* shifts in %. | | | |

**Table 3. P₅₀ values of whole blood after incubation with compounds 4,5,7,11-14 and 15, in vitro**

| Compound | *P₅₀* (Torr) whole blood | *P₅₀* (Torr) compound + whole blood | *P₅₀* increase (%) + SD |
|---|---|---|---|
| **4** | **(H)** 22.1 | 24.3 | 10 ± 4 |
| | **(M)** 37.9 | 42.7 | 13 ± 2 |
| **5** | **(H)** 22.1 | 30.8 | 39^{a} ± 5 |
| | **(P)** 31.6 | 44.2 | 40^{a} ± 3 |
| | **(M)** 36.7 | 47.4 | 29^{b} ± 3 |
| | **(M)** 40.1 | 52.0 | 30 ± 3 |
| **7** | **(M) 3**7.9 | 45.5 | 20 ± 2 |
| **11** | **(M)** 37.9 | 41.3 | 9 ± 1 |
| **12** | **(M)** 37.9 | 41.7 | 10 ± 2 |
| **13** | **(M)** 39.2 | 41.9 | 7 ± 1 |
| **14** | **(M)** 39.2 | 42.3 | 8 ± 2 |
| **15** | **(H)** 24.8 | 31.0 | 25 ± 3 |
| | **(M)** 40.1 | 55.3 | 38^{a} ± 4 |

| | | | |
|---|---|---|---|
| **H** = human; **M** = murine; **P** = porcine whole blood. Compound concentrations: **30**m**M**; means of (four single values) *P₅₀* shifts + **SD** are shown. ^{a} Compound concentration: **60** m**M**. ^{b} Compound concentration: **4** m**M**. | | | |

### REFERENCES

1. Fylaktakidou, K., Lehn, J.-M., Greferath, R., and Nicolau, C. (2004) Bioorg.Med.Chem. Lett (submitted)
2. Kim KJ, Li B, Winer J, Armanini M, Gillett N, Phillips HS, Ferrara N (1993) Nature 362, 841-844.
3. Kandel J, Bossy-Wetzel E, Radvanyi F, Klagsbrun M, Folkman J, Hanahan D (1991) Cell 66, 1095-1104.
4. O'Reilly MS, Boehm T, Shing Y, Fukai N, Vasios G, Lane WS, Flynn E, Birkhead JR, Olsen BR, Folkman J (1997) Cell 88, 277-285.
5. Good DJ, Polverini PJ, Rastinejad F, Le Beau MM, Lemons RS, Frazier WA, Bouck NP. (1990) Proc Natl Acad Sci USA 87, 6624-6628.
6. O'Reilly MS, Holmgren L, Shing Y, Chen C, Rosenthal RA, Moses M, Lane WS, Cao Y, Sage EH, Folkman J (1994) Cell 79, 315-328.
7. Chen C, Parangi S, Tolentino MJ, Folkman J. (1995) Cancer Res. 55, 4230-4233.
8. Ferrara N. (2002) Nat. Rev. Cancer 2, 795-803.
9. Ferrara N, Davis-Smyth T (1997) Endocr Rev. 18, 4-25.
10. Ferrara N, Gerber HP, LeCouter J. (2003) Nat Med. 9, 669-676.
11. Fontanini G, Vignati S, Boldrini L, Chine S, Silvestri V, Lucchi M, Mussi A, Angeletti CA, Bevilacqua G. (1997) Clin Cancer Res. 3, 861-865.
12. Dor Y, Porat R, Keshet E. (2001) Am J Physiol Cell Physiol. 280, C1367-1374.
13. Brizel DM, Scully SP, Harrelson JM, Layfield LJ, Bean JM, Prosnitz LR, Dewhirst MW (1996) Cancer Res. 56, 941-943.

## Claims

1. A pharmaceutical composition comprising the mixture of the sodium and calcium salt of myo-inositol tripyrophosphate and a pharmaceutically acceptable adjuvant, diluent, carrier, or excipient thereof, for use in the treatment of cancer.

2. The composition for use of claim 1, wherein the myo-inositol tripyrophosphate is myoinositol 1,6:2,3:4,5 tripyrophosphate.

3. The composition for use of claim 1 or claim 2, wherein the cancer is selected from the group consisting of rhabdomyosarcomas, retinoblastoma, Ewing's sarcoma, neuroblastoma, and osteosarcoma.

4. The composition for use of claim 1 or claim 2, wherein the cancer is selected from one or more of the group consisting of breast cancer, prostate cancer, renal cell cancer, brain cancer, ovarian cancer, colon cancer, bladder cancer, pancreatic cancer, stomach cancer, esophageal cancer, cutaneous melanoma, liver cancer, lung cancer, testicular cancer, kidney cancer, bladder cancer, cervical cancer, lymphoma, parathyroid cancer, penile cancer, rectal cancer, small intestine cancer, thyroid cancer, uterine cancer, Hodgkin's lymphoma, lip and oral cancer, skin cancer, leukemia, and multiple myeloma.

5. The composition for use of claim 2, wherein the dosage of myo-inositol 1,6:2,3:4,5 tripyrophosphate is 0.5 to 1.5 g/kg.

6. The pharmaceutical composition for use of any one of claims 1 to 5, wherein the composition is formulated for intravenous administration.

7. The composition for use of any one of Claims 1 to 6, wherein the mixture of the sodium and calcium salt of myo-inositol tripyrophosphate is obtained by mixing myo-inositol tripyrophosphate-sodium salt with CaCl₂.

## Patentansprüche

1. Eine pharmazeutische Zusammensetzung umfassend der Mischung aus dem Natrium- und Calciumsalz von Myo-Inositoltripyrophosphat und einem pharmazeutisch annehmbaren Adjuvans, Verdünnungsmittel, Träger, oder Hilfsstoff davon zur Verwendung in der Behandlung von Krebs.

2. Die Zusammensetzung zur Verwendung nach Anspruch 1, wobei das Myo-Inositoltripyrophosphat Myo-1,6:2,3:4,5-Inositoltripyrophosphat ist.

3. Die Zusammensetzung zur Verwendung nach Anspruch 1 oder Anspruch 2, wobei der Krebs ausgewählt ist aus der Gruppe, bestehend aus Rhabdomyosarkome, Retinoblastom, Ewing-Sarkom, Neuroblastom und Osteosarkom.

4. Die Zusammensetzung zur Verwendung nach Anspruch 1 oder Anspruch 2, wobei der Krebs ausgewählt ist von ein oder mehr aus der Gruppe bestehend aus Brustkrebs, Prostatakrebs, Nierenzellkarzinom, Hirntumor, Eierstockkrebs, Kolonkrebs, Blasenkrebs, Bauchspeicheldrüsenkrebs, Magenkrebs, Speiseröhrenkrebs, Hautmelanom, Leberkrebs, Lungenkrebs, Hodenkrebs, Nierenkrebs, Blasenkrebs, Gebärmutterhalskrebs, Lymphom, Nebenschilddrüsenkrebs, Peniskrebs, Rektumkarzinom, Dünndarmkrebs, Schilddrüsenkrebs, Gebärmutterkrebs, Hodgekin-Lymphom, Lippen- und Mundkrebs, Hautkrebs, Leukämie und Multiples Myelom.

5. Die Zusammensetzung zur Verwendung nach Anspruch 2, wobei die Dosierung von Myo-1,6:2,3:4,5-Inositoltripyrophosphat 0,5 bis 1,5 g/kg ist.

6. Die Zusammensetzung zur Verwendung nach einem der Ansprüche 1 bis 5, wobei die Zusammensetzung zur intravenösen Verabreichung formuliert ist.

7. Die Zusammensetzung zur Verwendung nach einem der Ansprüche 1 bis 6, wobei die Mischung aus dem Natrium- und Calciumsalz von Myo-Inositoltripyrophosphat durch Mischen von Myo-Inositoltripyrophosphat-Natriumsalz mit CaCl₂ erhalten wird.

## Revendications

1. Composition pharmaceutique comprenant le mélange du sel de sodium et de calcium de tripyrophosphate de myo-inositol et d'un adjuvant, diluant, support ou excipient pharmaceutiquement acceptable de celui-ci, pour son utilisation dans le traitement du cancer.

2. Composition pour son utilisation selon la revendication 1, dans laquelle le tripyrophosphate de myo-inositol est le 1,6:2,3:4,5-tripyrophosphate de myo-inositol.

3. Composition pour son utilisation selon la revendication 1 ou la revendication 2, dans laquelle le cancer est choisi dans le groupe constitué de rhabdomyosarcomes, rétinoblastome, sarcome de Ewing, neuroblastome, et ostéosarcome.

4. Composition pour son utilisation selon la revendication 1 ou la revendication 2, dans laquelle le cancer est choisi parmi un ou plusieurs du groupe constitué du cancer du sein, du cancer de la prostate, du cancer des cellules rénales, du cancer du cerveau, du cancer ovarien, du cancer du côlon, du cancer de la vessie, du cancer pancréatique, du cancer de l'estomac, du cancer oesophagien, du mélanome cutané, du cancer du foie, du cancer du poumon, du cancer testiculaire, du cancer du rein, du cancer de la vessie, du cancer du col de l'utérus, du lymphome, du cancer parathyroïdien, du cancer pénien, du cancer rectal, du cancer de l'intestin grêle, du cancer de la thyroïde, du cancer de l'utérus, du lymphome de Hodgkin, du cancer de la lèvre et buccal, du cancer de la peau, de la leucémie, et du myélome multiple.

5. Composition pour son utilisation selon la revendication 2, dans laquelle le dosage du 1,6:2,3:4,5-tripyrophosphate de myo-inositol est de 0,5 à 1,5 g/kg.

6. Composition pharmaceutique pour son utilisation selon l'une quelconque des revendications 1 à 5, dans laquelle la composition est formulée pour une administration intraveineuse.

7. Composition pour son utilisation selon l'une quelconque des revendications 1 à 6, dans laquelle le mélange du sel de sodium et de calcium du tripyrophosphate de myo-inositol est obtenu par mélange du sel de sodium de tripyrophosphate de myo-inositol avec du CaCl₂.
